(19) Europäisches Patentamt
European Patent Office
Office européen des brevets

(11) **EP 2 638 806 A1**

(12) **EUROPEAN PATENT APPLICATION**
published in accordance with Art. 153(4) EPC

(43) Date of publication:
18.09.2013 Bulletin 2013/38

(21) Application number: 11839755.3

(22) Date of filing: 08.11.2011

(51) Int Cl.:
*A23D 9/007* (2006.01)    *A23K 1/16* (2006.01)
*A23L 1/30* (2006.01)    *C07J 13/00* (2006.01)
*A23K 1/00* (2006.01)

(86) International application number:
**PCT/JP2011/075662**

(87) International publication number:
**WO 2012/063794 (18.05.2012 Gazette 2012/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 09.11.2010 JP 2010250918

(71) Applicant: Tsuno Food Industrial Co., Ltd.
Ito-gun
Wakayama 649-7194 (JP)

(72) Inventors:
• **TSUNO, Takuo**
**Ito-gun**
**Wakayama 649-7194 (JP)**

• **YAMANAKA, Takashi**
**Ito-gun**
**Wakayama 649-7194 (JP)**
• **SEGOSHI, Hiroaki**
**Ito-gun**
**Wakayama 649-7194 (JP)**

(74) Representative: **Keller, Günter et al**
**Lederer & Keller**
**Patentanwälte**
**Unsöldstrasse 2**
**80538 München (DE)**

(54) **METHOD FOR PRODUCING OIL OR FAT THAT CONTAINS γ-ORYZANOL**

(57) The disclosed method for producing a γ-oryzanol-containing fat or oil, the method comprising the steps of:
(A) preparing a processed fat or oil having an oil layer and a soapstock (alkali-foots) layer containing a γ-oryzanol salt; and
(B) adding an acid to the processed fat or oil of step (A) for transfer of γ-oryzanol from the soapstock layer to the oil layer, and collecting a fat or oil with an increased γ-oryzanol content, enables inexpensive production of a fat or oil containing γ-oryzanol at any desired concentration without restriction to the γ-oryzanol concentration in the raw fat or oil.

Printed by Jouve, 75001 PARIS (FR)

**Description**

TECHNICAL FIELD

The present invention relates to a method for producing a γ-oryzanol-containing fat or oil, and a γ-oryzanol-containing fat or oil producible by the method.

BACKGROUND ART

γ- Oryzanol is a group of esters of triterpene alcohols or various plant sterols with ferulic acid, and is abundantly present as a bioactive component in rice bran used as raw material of rice bran oil. γ- Oryzanol, for example in the case of use for external preparations, has been reported to exhibit antioxidant effect, ultraviolet absorbing effect, inhibitory effect on tyrosinase activity, skin temperature elevating effect, cutaneous gland stimulating effect and the like. γ- Oryzanol has also been reported to have growth promoting effect, gonadotropic effect, vitamin E- like effect, antistress effect, decholesterolizing effect, hypolipidemic effect and the like as a pharmacological effect. For these reasons, γ- oryzanol is widely used as a food additive, a cosmetic material, a pharmaceutical material, a dietary supplement, a feed ingredient and the like.

Generally, rice bran oil is produced by separating crude oil by hexane extraction from rice bran used as raw material, and processing the crude oil into refined rice bran oil through the steps of degumming, dewaxing, deacidification, decolorization and deodorization. In the deacidification step, alkali deacidification, which allows free fatty acids in the crude oil to be removed as a soapstock resulting from neutralization with an alkali (caustic soda) , is usually used. In this case, a large part of γ- oryzanol present in an amount of about 2 mass% in the crude oil is transferred to the soapstock and removed.

Therefore, for high retention of γ- oryzanol, distillative deacidification, which allows physical removal of free fatty acids by steam distillation, is proposed (for example, see Patent Literature 1 and 2) . Also, deacidification with a weak alkali is proposed for high retention of γ- oryzanol (for example, see Patent Literature 3) . Further, implementation of a dehydration step before removal of oil foots resulting from the ordinary alkali deacidification is proposed for high retention of γ- oryzanol (for example, see Patent Literature 4) .

CITATION LIST

Patent Literature

    Patent Literature 1: JP-A 6-340889
    Patent Literature 2: JP-A 2002-238455
    Patent Literature 3: JP-A 2000-119682
    Patent Literature 4: JP-A 2009-108145

SUMMARY OF INVENTION

TECHNICAL PROBLEM

The disclosure in Patent Literature 1 to 4 is all relevant to a technique for efficient retention of the original γ-oryzanol content of the raw fat or oil, and there is no disclosure of techniques for producing a fat or oil containing γ-oryzanol at a concentration equal to or higher than that in the raw fat or oil. There is also no disclosure of techniques for producing a γ-oryzanol-containing fat or oil from an originally γ-oryzanol-free fat or oil.

A fat or oil containing γ-oryzanol at any desired concentration can be produced by external addition of γ-oryzanol, but in Japan, γ-oryzanol as a food additive is approved only for use as an anti-oxidant, and due to such a restriction, cannot be indicated as a nutritional ingredient. Further, γ-oryzanol is so expensive (about 10000 yen/kg) that addition of 2 mass% of γ-oryzanol to fat or oil, for example, results in cost increase by about 200 yen/kg.

Therefore, an object of the present invention is to provide a method for inexpensively producing a γ- oryzanol-rich fat or oil containing γ- oryzanol at any desired concentration without restriction to the γ- oryzanol concentration in the raw fat or oil.

SOLUTION TO PROBLEM

The present invention includes the following as a solution to the above-mentioned problems.

(1) A method for producing a γ-oryzanol-containing fat or oil, the method comprising the steps of:

(A) preparing a processed fat or oil having an oil layer and a soapstock (alkali- foots) layer containing a γ-oryzanol salt, and
(B) adding an acid to the processed fat or oil of step (A) for transfer of γ-oryzanol from the soapstock layer to the oil layer, and collecting a fat or oil with an increased γ-oryzanol content.

(2) The method according to the above (1), wherein, in step (A), the processed fat or oil is prepared by superposing a fat or oil onto a soapstock containing a γ-oryzanol salt.
(3) The method according to the above (1), wherein, in step (A), the processed fat or oil is prepared by adding an alkali to a raw fat or oil containing γ-oryzanol for separation of an oil layer and a soapstock layer containing a γ-oryzanol salt.
(4) The method according to the above (3), further comprising removing part of the oil layer from the processed fat or oil.
(5) The method according to the above (3), further comprising removing part of the oil layer from the processed fat or oil, and freshly adding a fat or oil to the remaining oil layer.
(6) The method according to any one of the above (3) to (5), wherein the raw fat or oil is a rice bran-derived fat or oil.
(7) The method according to any one of the above (1) to (6), wherein the pH of the soapstock layer containing a γ-oryzanol salt is higher than 10 in step (A).
(8) The method according to any one of the above (1) to (7), wherein, in step (B), the acid is added in such an amount that the pH of the soapstock layer becomes 10 or lower.
(9) The method according to the above (8), wherein, in step (B), the acid is added in such an amount that the pH of the soapstock layer becomes 7 to 10.
(10) The method according to any one of the above (1) to (9), wherein the acid is one or more kinds selected from the group consisting of hydrochloric acid, sulfuric acid, nitric acid, acetic acid, phosphoric acid, citric acid, lactic acid, malic acid, phytic acid, gluconic acid, tartaric acid, butyric acid, oxalic acid, fatty acid and deodorizer distillate.
(11) The method according to any one of the above (1) to (10), further comprising a decolorization step and a deodorization step after step (B).
(12) A γ-oryzanol-containing fat or oil producible by the method according to any one of the above (1) to (11).
(13) The γ-oryzanol-containing fat or oil according to the above (12), containing 2 mass% or more of γ-oryzanol.
(14) The γ-oryzanol-containing fat or oil according to the above (12) or (13), which is one or more kinds selected from the group consisting of rice bran oil, palm oil, rapeseed oil, soybean oil, sesame oil, corn oil, cotton seed oil, coconut oil, olive oil, palm oil, safflower oil, sunflower seed oil, almond oil, cashew oil, hazelnut oil, macadamia nut oil, mongongo oil, pecan oil, pine nut oil, walnut oil, camellia oil, tea seed oil, beef tallow, lard, chicken oil, horse oil and fish oil.
(15) The γ-oryzanol-containing fat or oil according to the above (12) or (13), which is one or more kinds selected from the group consisting of mineral oil, squalene, lanolin, isopropyl isostearate, isostearyl isostearate, octyl isononanoate, ethyl oleate, ethyl oleate, octyl stearate, stearyl stearate, octyl palmitate, isopropyl palmitate, isopropyl myristate and octyldodecyl myristate.

ADVANTAGEOUS EFFECTS OF INVENTION

The production method of the present invention enables inexpensive production of a γ-oryzanol-rich fat or oil containing γ-oryzanol at any desired concentration without restriction to the γ-oryzanol concentration in the raw fat or oil. The fat or oil obtainable by the production method of the present invention is not relevant to external addition of γ-oryzanol, and therefore γ-oryzanol in such a fat or oil is exempted from Japan's restrictions on food additives and can be indicated as a nutritional ingredient.

BRIEF DESCRIPTION OF DRAWINGS

Fig. 1 shows the results of the examination for oxidation stability of γ-oryzanol-rich rice bran oils.
Fig. 2 shows the results of the examination for oxidation stability of γ-oryzanol-containing palm oils.

DESCRIPTION OF EMBODIMENTS

The method of the present invention for producing a γ-oryzanol-containing fat or oil (hereinafter referred to as "the production method of the present invention") comprises the steps of:

(A) preparing a processed fat or oil having an oil layer and a soapstock (alkali- foots) layer containing a γ- oryzanol salt, and
(B) adding an acid to the processed fat or oil of step (A) for transfer of γ-oryzanol from the soapstock layer to the oil layer, and collecting a fat or oil with an increased γ-oryzanol content.

The production method of the present invention may further comprise a step other than steps (A) and (B) unless the objects of the present invention are impaired. Examples of the step other than steps (A) and (B) include a decolorization step and a deodorization step. Preferably, the decolorization step and the deodorization step are implemented after steps (A) and (B) . Through the decolorization step and the deodorization step, a γ-oryzanol-containing refined fat or oil suitable as a marketed product can be obtained. In the decolorization step and the deodorization step, known decolorization methods and deodorization methods which are usually used in the production process of edible fats and oils can be used.
The processed fat or oil of step (A) is not particularly limited as long as it has an oil layer and a soapstock layer containing a γ-oryzanol salt. The processed fat or oil may consist of only an oil layer and a soapstock layer, or consist of the two layers and another layer. In step (A), the processed fat or oil can be prepared by adding an alkali to a raw fat or oil containing γ-oryzanol for separation of an oil layer and a soapstock layer. The raw fat or oil is not particularly limited and may be any fat or oil that contains γ-oryzanol. γ-Oryzanol is abundantly present in rice bran, and thus a rice bran-derived fat or oil is preferable as the raw fat or oil. Corn oil is also known to contain γ-oryzanol, and thus a corn germ-derived fat or oil can be used as the raw fat or oil.
As mentioned above, a crude oil separated by hexane extraction from rice bran used as raw material (hereinafter referred to as "crude rice bran oil") contains about 2 mass% of γ-oryzanol, and a large part of the γ-oryzanol present in the crude oil is removed by alkali deacidification in the deacidification step. Therefore, a crude rice bran oil that is not yet subjected to alkali deacidification in the deacidification step can be preferably used as the raw fat or oil. Specific examples of such a crude rice bran oil include a crude rice bran oil just separated by hexane extraction, a crude rice bran oil processed through the degumming step, and a crude rice bran oil processed through the degumming step and the dewaxing step. Since the removal of γ-oryzanol by alkali deacidification in the deacidification step is based on transfer of γ-oryzanol to the soapstock, a fat or oil obtainable by acidolysis of the soapstock (also called acidulated soapstock or acid oil) can be preferably used as the raw fat or oil.
The alkali added to the raw fat or oil is not particularly limited, but a strong alkali is preferable in terms of efficiency in deacidification and decolorization. Examples of the strong alkali include caustic soda (sodium hydroxide) and caustic potash (potassium hydroxide). After the alkali addition to the raw fat or oil, the mixture is stirred, allowed to stand, and subjected to centrifugation or the like, resulting in separation of an oil layer and a soapstock layer. The alkali treatment temperature is not particularly limited, but is usually about 20 to 80°C, and preferably about 30 to 50°C. The alkali treatment time is not particularly limited either, but is usually about 15 minutes to 2 hours, and preferably about 30 minutes to 1 hour. γ-Oryzanol present in the raw fat or oil is neutralized with the alkali to form a salt, and transferred to the soapstock layer. In addition to the alkali, another substance may be added to the raw fat or oil unless the objects of the present invention are impaired. Examples of such a substance include, but are not limited to, solvents such as hexane, ethanol, isopropanol and methanol.
The amount of the alkali added to the raw fat or oil is not particularly limited, but in the case where the raw fat or oil contains free fatty acid, it is preferable to add the alkali in an equivalent or excess amount relative to the acid value of the free fatty acid. It is more preferable to add about 1.2 Eq or more of the alkali relative to the acid value of the free fatty acid. The maximum amount of the alkali that can be added is not particularly limited, but about 2 to 4 Eq should be enough. The acid value of the free fatty acid can be determined by, for example, titration using alkali blue as an indicator according to the method described in "The Standard Methods for the Analysis of Fats, Oils and Related Materials, 2003 Edition (compiled by Japan Oil Chemists' Society)."
The amount of the alkali added to the raw fat or oil is preferably such that the pH of the soapstock becomes higher than 10. When the pH of the soapstock becomes higher than 10, a larger part of the γ-oryzanol present in the raw fat or oil forms a salt and is expected to readily transfer to the soapstock layer. The maximum amount of the alkali that can be added is not particularly limited, but the amount that makes the pH about 12 to 14 should be enough.
In step (A), the processed fat or oil having an oil layer and a soapstock layer containing a γ-oryzanol salt can also be prepared by superposing a fat or oil onto a soapstock containing a γ-oryzanol salt. For example, a soapstock produced as waste in the production of rice bran oil is collected, a fat or oil is superposed onto the soapstock, and thereby the processed fat or oil having an oil layer and a soapstock layer containing a γ-oryzanol salt can be prepared. As an alternative, the following procedures may be performed: after alkali addition to a raw fat or oil containing γ-oryzanol, the mixture is stirred, allowed to stand and optionally subjected to any other operation, the resulting oil layer is entirely removed, and a fat or oil is freshly superposed onto the remaining soapstock layer. As another alternative, the following procedures may be performed: after alkali addition to a raw fat or oil containing γ-oryzanol, the mixture is stirred, allowed to stand and optionally subjected to any other operation, the resulting oil layer is partially removed, and a fat or oil is freshly added to the remaining oil layer.

In step (B), an acid is added to the processed fat or oil of step (A), which has an oil layer and a soapstock layer, for transfer of γ-oryzanol from the soapstock layer to the oil layer. The acid is not particularly limited and examples thereof include hydrochloric acid, sulfuric acid, nitric acid, acetic acid, phosphoric acid, citric acid, lactic acid, malic acid, phytic acid, gluconic acid, tartaric acid, butyric acid, oxalic acid, fatty acid and deodorizer distillate. These acids may be used alone or in a combination of two kinds or more. Inter alia, preferred are sulfuric acid, phosphoric acid, fatty acid and deodorizer distillate, and more preferred are sulfuric acid, phosphoric acid and deodorizer distillate. Deodorizer distillate is produced as waste in the deodorization step in edible fat and oil production, and is also called scum oil. Deodorizer distillate is rich with tocopherols and sterols in addition to free fatty acids, and use of deodorizer distillate as the acid can increase tocopherol and sterol contents of the γ-oryzanol-containing fat or oil obtainable by the production method of the present invention.

After the acid addition to the processed fat or oil having an oil layer and a soapstock layer, the mixture is stirred, allowed to stand, and subjected to centrifugation or the like, resulting in separation of an oil layer and a soapstock layer. Then, only the upper layer, i.e. the oil layer with an increased γ-oryzanol content is collected. The acid treatment temperature is not particularly limited, but is usually about 60 to 100°C, and preferably about 70 to 90°C. The acid treatment time is not particularly limited either, but is usually about 15 minutes to 2 hours, and preferably about 30 minutes to 1 hour. For removal of as much soap as possible from the collected oil layer, the following procedures may be performed: water is added to the collected oil layer, the mixture is stirred and the resulting aqueous layer (soap) is removed.

The amount of the acid added in step (B) is not particularly limited, but is preferably such that the pH of the soapstock layer becomes 10 or lower. That is, it is preferable to add the acid in such an amount that the pH of the soapstock layer becomes 10 or lower in the separation of an oil layer and a soapstock layer after acid addition and stirring. When the pH of the soapstock layer becomes 10 or lower, the γ-oryzanol salt present in the soapstock layer is converted into the free form of γ-oryzanol and expected to readily transfer to the oil layer. The maximum amount of the acid that can be added in step (B) is not particularly limited, and for example, the acid may be added in such an amount that the pH of the soapstock layer becomes about 1 to 3. However, considering that the acidic pH of the soapstock layer causes fatty acids to be released from free fatty acid salts (soap) in the soapstock layer and transferred to the oil layer, the amount of the acid added in step (B) is preferably such that the pH of the soapstock layer becomes 3 to 10, more preferably 4 to 10, further more preferably 5 to 10, further more preferably 6 to 10, and particularly preferably 7 to 10.

Since step (B) allows a large part of γ-oryzanol to be recovered from the soapstock layer and transferred to the oil layer, for example in the case where a crude rice bran oil containing about 2 mass% of γ-oryzanol is used as the raw fat or oil, a rice bran oil containing about 2 mass% of γ-oryzanol can be produced. According to the techniques described in Patent Literature 1 to 4 for efficient retention of the original γ-oryzanol content of the raw fat or oil, the amount of γ-oryzanol retained in refined rice bran oil is at most about 1.4 mass%, and unlikely to reach about 2 mass%.

In the case where the processed fat or oil of step (A) is prepared by adding an alkali to a raw fat or oil containing γ-oryzanol for separation of an oil layer and a soapstock layer containing a γ-oryzanol salt, removing part of the oil layer enables production of a fat or oil containing γ-oryzanol at a higher concentration than that in the raw fat or oil. In this case, changing the removal ratio of the oil layer as appropriate enables production of a γ-oryzanol-rich fat or oil containing γ-oryzanol at any desired concentration. In the case where the processed fat or oil of step (A) is prepared by adding an alkali to a raw fat or oil containing γ-oryzanol for separation of an oil layer and a soapstock layer containing a γ-oryzanol salt, and subsequently part of the oil layer is removed, a fat or oil may be freshly added to the remaining oil layer. In this case, adding different kinds of fats or oils enables production of a blend fat or oil containing γ-oryzanol at any desired concentration.

In the case where the processed fat or oil of step (A) is prepared by superposing a fat or oil onto a soapstock containing a γ-oryzanol salt, using an originally γ-oryzanol-free fat or oil or a fat or oil with a low γ-oryzanol content as the superposed fat or oil enables production of any desired kind of γ-oryzanol-containing fat or oil. On the other hand, using a γ-oryzanol-containing fat or oil as the superposed fat or oil enables production of a fat or oil containing γ-oryzanol at a further higher concentration. Further, repeatedly using the thus-obtained γ-oryzanol-rich fat or oil as the superposed fat or oil enables production of a fat or oil containing γ-oryzanol at a very high concentration.

The γ-oryzanol-containing fat or oil obtainable by the production method of the present invention is preferably a refined edible fat or oil without any externally added γ-oryzanol. Examples of such an edible fat or oil include rice bran oil, palm oil, rapeseed oil, soybean oil, sesame oil, corn oil, cotton seed oil, coconut oil, olive oil, palm oil, safflower oil, sunflower seed oil, almond oil, cashew oil, hazelnut oil, macadamia nut oil, mongongo oil, pecan oil, pine nut oil, walnut oil, camellia oil, tea seed oil, beef tallow, lard, chicken oil, horse oil and fish oil. Two or more of the foregoing may be mixed to give a blend oil. The γ-oryzanol content of the γ-oryzanol-containing fat or oil is not particularly limited, but for example in the case of rice bran oil, the γ-oryzanol content is preferably 0.5 mass% or more, more preferably 2 mass% or more, further preferably 5 mass% or more, further preferably 10 mass% or more, and particularly preferably 30 mass% or more. In the case of edible fats and oils (including a blend oil) other than rice bran oil, the γ-oryzanol content may be any amount that is measurable, but is preferably 0.1 mass% or more, more preferably 0.5 mass% or more, further preferably 2 mass% or more, further preferably 5 mass% or more, further preferably 10 mass% or more, and further preferably 30 mass% or more. The maximum limit of the γ-oryzanol content is not particularly limited, and according to the production method of the present invention, a fat or oil containing as much as about 60 mass% of γ-oryzanol can be produced. The γ-oryzanol concentration in fats or oils can be measured according to a known method, for example, the method described in Examples.

Examples of the above-mentioned fat or oil freshly added after removal of the oil layer in the production method of the present invention also include fats and oils used as a cosmetic material, such as mineral oil, squalene and lanolin; and ester oils, such as isopropyl isostearate, isostearyl isostearate, octyl isononanoate, ethyl oleate, ethyl oleate, octyl stearate, stearyl stearate, octyl palmitate, isopropyl palmitate, isopropyl myristate and octyldodecyl myristate, and with the use of the foregoing, a γ-oryzanol-containing fat or oil usable as a cosmetic material can be obtained. The γ-oryzanol content of the obtained fat or oil is not particularly limited as long as it is a measurable amount, but the γ-oryzanol content is preferably 0.1 mass% or more, more preferably 0.5 mass% or more, further preferably 2 mass% or more, further preferably 5 mass% or more, further preferably 10 mass% or more, and further preferably 30 mass% or more.

EXAMPLES

Hereinafter, the present invention will be illustrated in detail by Examples, but is not limited thereto.

<Example 1: Production of γ-oryzanol-rich rice bran oil>

(1) Step (A)

A hundred kilograms of a rice bran oil processed through the degumming step and the dewaxing step (acid value: 4.3) was heated to 40°C, 1.69 kg of a 25.5 mass% caustic soda aqueous solution and 3.37 kg of water were added, and the mixture was stirred for 30 minutes, resulting in separation of an oil layer and a soapstock layer.

(2) Step (B)

To the product obtained above, 0.82 kg of a 75% phosphoric acid aqueous solution was added, and the mixture was heated to 80°C with stirring. After centrifugation, an oil layer (acid value: 1.6) weighing 90 kg was obtained. To the obtained oil layer, warm water at 40°C was added, and the mixture was centrifuged again for removal of soap. In this way, an acid-treated oil was obtained.

(3) Decolorization step

To the acid-treated oil, 4.5 kg of activated clay was added, and the mixture was heated to 120°C at 8000 Pa. After filtration, a decolorized oil was obtained.

(4) Deodorization step

The decolorized oil was heated to 240°C at 1300 Pa for removal of odor components, and finally an oryzanol-rich rice bran oil (acid value: 0.3) was obtained.

The weight, acid value and γ-oryzanol concentration of the oil or oil foots obtained in each step are shown in Table 1. Quantification of γ-oryzanol and determination of the acid value were performed by the following respective methods.

(a) Quantification of γ-oryzanol

Two to Five grams of fat or oil was weighed out and n-hexane was added to make a total volume of 100 mL. Two milliliters of this solution was diluted with n-hexane to make a total volume of 100 mL, and the absorbance of the diluted solution was measured at 315 nm. When the weight of the fat or oil and the absorbance are expressed as X (g) and A, respectively, the γ-oryzanol content expressed as G (%) can be determined by the following formula.

$$G = (A \times 5000)/(X \times 359)$$

(b) Determination of acid value

The acid value was determined by titration according to the method described in "The Standard Methods for the Analysis of Fats, Oils and Related Materials, 2003 Edition (compiled by Japan Oil Chemists' Society)." In the case of γ-oryzanol-containing oils, alkali blue was used as an indicator.

Table 1

|  | Weight (kg) | Acid value | OZ concentration (mass%) |
|---|---|---|---|
| Dewaxed rice bran oil | 100 | 4.3 | 2.0 |
| Oil layer of step (A) | 90 | 0 | 0.3 |
| Alkali foots | 10 | - | 36.0 |
| Acid-treated oil | 90 | 1.6 | 1.7 |
| OZ-rich rice bran oil | 80 | 0.3 | 1.6 |
| OZ: γ-Oryzanol | | | |

<Example 2: Production of γ-oryzanol-rich rice bran oil>

(1) Step (A)

A hundred kilograms of a rice bran oil processed through the degumming step and the dewaxing step (acid value: 4.3) was heated to 40°C, 1.69 kg of a 25.5 mass% caustic soda aqueous solution and 3.37 kg of water were added, and the mixture was stirred for 30 minutes, resulting in separation of an oil layer and a soapstock layer.

(2) Step (B)

Seventy kilograms of the oil layer was removed, 0.82 kg of a 75% phosphoric acid aqueous solution was added to the remaining oil layer (acid value: 0) and the soapstock layer, and the mixture was heated to 80°C with stirring. After centrifugation, an oil layer (acid value: 1.6) weighing 20 kg was obtained. To the obtained oil layer, warm water at 40°C was added, and the mixture was centrifuged again for removal of soap. In this way, an acid-treated oil was obtained.

(3) Decolorization step

To the acid-treated oil, 1 kg of activated clay was added, and the mixture was heated to 120°C at 8000 Pa. After filtration, a decolorized oil was obtained.

(4) Deodorization step

The decolorized oil was heated to 240°C at 1300 Pa for removal of odor components, and finally an oryzanol-rich rice bran oil (acid value: 0.3) was obtained.
The weight, acid value and γ-oryzanol concentration of the oil or oil foots obtained in each step are shown in Table 2.

Table 2

|  | Weight (kg) | Acid value | OZ concentration (mass%) |
|---|---|---|---|
| Dewaxed rice bran oil | 100 | 4.3 | 2.0 |
| Oil layer of step (A) | 90 | 0 | 0.3 |
| Alkali foots | 10 | - | 36.0 |
| Acid-treated oil | 20 | 1.6 | 6.2 |

(continued)

|  | Weight (kg) | Acid value | OZ concentration (mass%) |
|---|---|---|---|
| OZ-rich rice bran oil | 19 | 0.3 | 6.0 |
| OZ: γ-Oryzanol | | | |

<Example 3: Production of γ-oryzanol-containing palm oil>

(1) Step (A)

A thousand grams of a rice bran oil processed through the degumming step and the dewaxing step (acid value: 4.3) was heated to 40°C, 16.86 g of a 25.5 mass% caustic soda aqueous solution and 33.73 g of water were added, and the mixture was stirred for 30 minutes, resulting in separation of an oil layer and a soapstock layer.

(2) Step (B)

The oil layer was entirely removed, 1000 g of RBD palm oil (acid value: 0) and 40 g of deodorizer distillate (scum oil) (acid value: 83.3) were successively added to 120 g of the remaining soapstock layer, and the mixture was heated to 80°C with stirring. After centrifugation, an oil layer (acid value: 1.7) weighing 1040 g was obtained. To the obtained oil layer, warm water at 40°C was added, and the mixture was centrifuged again for removal of soap. In this way, an acid-treated oil was obtained.

(3) Decolorization step

To the acid-treated oil, 20 g of activated clay was added, and the mixture was heated to 120°C at 8000 Pa. After filtration, a decolorized oil was obtained.

(4) Deodorization step

The decolorized oil was heated to 240°C at 1300 Pa for removal of odor components, and finally an oryzanol-containing palm oil (acid value: 0.4) was obtained.

The weight, acid value and γ-oryzanol concentration of the oil or oil foots obtained in each step are shown in Table 3.

Table 3

|  | Weight (g) | Acid value | OZ concentration (mass%) |
|---|---|---|---|
| Dewaxed rice bran oil | 1000 | 4.3 | 2.0 |
| Oil layer of step (A) | 929 | 0 | 0.3 |
| Alkali foots | 120 | - | 36.0 |
| Palm oil | 1000 | 0 | - |
| Scum oil | 40 | 83.3 | - |
| Acid-treated oil | 1040 | 1.7 | 1.5 |
| OZ-containing palm oil | 856 | 0.4 | 1.4 |
| OZ: γ-Oryzanol | | | |

<Example 4: Production of γ-oryzanol-containing rapeseed oil>

The same procedures as described in Example 3 were performed to give a rapeseed oil containing 1. 5 mass% of γ-oryzanol, except that a RBD rapeseed oil processed through the deacidification step (acid value: 0) was used as the oil added in step (B) instead of the RBD palm oil (acid value: 0).

<Example 5: Examination for oxidation stability of γ-oryzanol-rich rice bran oils>

The γ-oryzanol-rich rice bran oil of Example 1, the γ-oryzanol-rich rice bran oil of Example 2, and a standard rice bran oil (γ-oryzanol content: 0.1 mass%) were evaluated for oxidation stability in the CDM test described in "The Standard Methods for the Analysis of Fats, Oils and Related Materials, 2003 Edition (compiled by Japan Oil Chemists' Society)." Specifically, the CDM test was performed using Rancimat model 743, an automatic oxidation stability tester for fats and oils (manufactured by Metrohm Japan; and compliant with the CDM test of "The Standard Methods for the Analysis of Fats, Oils and Related Materials, 2003 Edition (compiled by Japan Oil Chemists' Society)") under the following conditions: the sample weight was 3 g; and the heating temperature was 120°C).

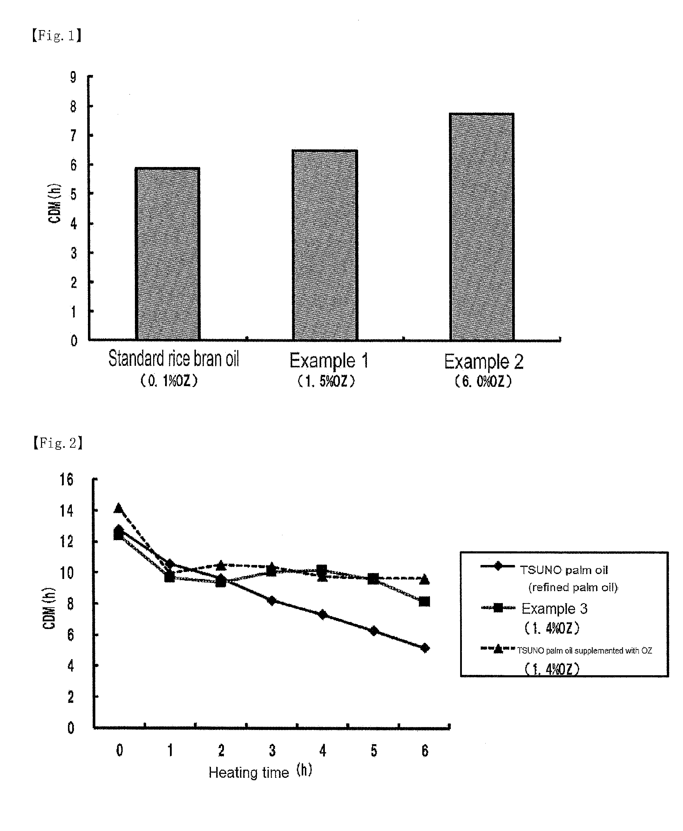

The results are shown in Fig. 1. Fig. 1 clearly shows that, as the γ-oryzanol concentration becomes higher, the CDM value increases and thus the oxidation stability increases.

<Example 6: Examination for oxidation stability of γ-oryzanol-containing palm oils>

TSUNO palm oil (refined palm oil), TSUNO palm oil supplemented with γ-oryzanol (manufactured by Tsuno Food Industrial Co., Ltd.) (γ-oryzanol content: 1.4 mass%), and the oryzanol-containing palm oil of Example 3 (γ-oryzanol content: 1.4 mass%) were subjected to a water spray test. That is, 500 g of each test oil was placed in an aluminum block bath and the oil temperature was kept at 180°C with relatively gentle stirring with a magnetic stirrer. After the oil temperature reached the predetermined temperature, from the nozzle of a glass spray fixed 5 cm above the oil surface, a fixed volume (80 mL/h) of distilled water supplied with a constant flow pump was sprayed while the air content was adjusted. About 10 mL of each test oil was sampled before the start of heating (0 hour) and once every hour for 6 hours of heating. The sampled test oil was encapsulated with nitrogen and stored in a refrigerator with protection from light until use in the CDM test. The CDM test was performed in the same manner as in Example 3.

The results are shown in Fig. 2. As is clear from Fig. 2, the CDM value of the originally γ-oryzanol-free TSUNO palm oil was reduced over time, but in the oryzanol-containing palm oil of Example 3 and the TSUNO palm oil supplemented with 1.4 mass% of γ-oryzanol, reduction in the CDM value was inhibited all the time after the first hour passed, thus indicating that the latter two oils have an improved oxidation stability and are less prone to degradation.

<Example 7: Change in aroma by heating>

A standard rice bran oil, the oryzanol-rich rice bran oil of Example 1 (γ-oryzanol content: 1.5 mass%), a rapeseed oil (manufactured by Okamura Oil Mill Co., Ltd.), and the oryzanol-containing rapeseed oil of Example 4 (γ-oryzanol content: 1.5 mass%) were subjected to a water spray test. Sensory evaluation was performed in terms of aroma of the oils at the time when the oil temperature reached 180°C (at the initiation of the test), and at 6 hours from the start of water spraying (at the end of the test).

The aroma was evaluated on a five-point scale as follows.

1: Intense sweet aroma
2: Mild sweet aroma
3: Aroma of oil at ordinary temperature
4: Mild unpleasant smell
5: Intense unpleasant smell

The results are shown in Table 4. As is shown in Table 4, there was no change in the aroma of the standard rice bran, but the oryzanol-rich rice bran oil of Example 1, after continuously heated, smelled sweet. The heated rapeseed oil had an intense unpleasant smell, but the unpleasant smell was reduced in the heated oryzanol-containing rapeseed oil of Example 4.

Table 4

| | Aroma evaluation (1 to 5) | |
| --- | --- | --- |
| | Initiation point | End point |
| Standard rice bran oil | 3 | 3 |
| Example 1 | 3 | 2 |
| Rapeseed oil | 4 | 5 |
| Example 4 | 4 | 3 |

<Example 8: Storage test>

The storage test was performed on the oryzanol-rich rice bran oil of Example 1 (γ-oryzanol content: 1.5 mass%), and a rice bran oil (γ-oryzanol content: 1.5 mass%) obtained by completely dissolving γ-oryzanol (manufactured by Tsuno Food Industrial Co., Ltd.) in a standard rice bran oil. That is, 100 g each of these two kinds of oil were charged into separate glass bottles, and the glass bottles were covered with lids and stored at 0°C for 24 hours.

As a result, there was no change in the appearance of the oryzanol-rich rice bran oil of Example 1, but in the standard rice bran oil supplemented with γ-oryzanol, crystals were observed. The analysis of the crystals revealed that the main component was γ-oryzanol. These results demonstrated that the oryzanol-containing oil obtained according to the production method of the present invention is more excellent in storage stability.

The present invention is not limited to particular embodiments and examples described above, and various modifications can be made within the scope of the appended claims. Other embodiments provided by suitably combining technical means disclosed in separate embodiments of the present invention are also within the technical scope of the present invention. All the academic publications and patent literatures cited in the above description are incorporated herein by reference.

**Claims**

1. A method for producing a γ-oryzanol-containing fat or oil, the method comprising the steps of:

   (A) preparing a processed fat or oil having an oil layer and a soapstock (alkali-foots) layer containing a γ-oryzanol salt, and
   (B) adding an acid to the processed fat or oil of step (A) for transfer of γ-oryzanol from the soapstock layer to the oil layer, and collecting a fat or oil with an increased γ-oryzanol content.

2. The method according to claim 1, wherein, in step (A), the processed fat or oil is prepared by superposing a fat or oil onto a soapstock containing a γ-oryzanol salt.

3. The method according to claim 1, wherein, in step (A), the processed fat or oil is prepared by adding an alkali to a raw fat or oil containing γ-oryzanol for separation of an oil layer and a soapstock layer containing a γ-oryzanol salt.

4. The method according to claim 3, further comprising removing part of the oil layer from the processed fat or oil.

5. The method according to claim 3, further comprising removing part of the oil layer from the processed fat or oil, and freshly adding a fat or oil to the remaining oil layer.

6. The method according to any one of claims 3 to 5, wherein the raw fat or oil is a rice bran-derived fat or oil.

7. The method according to any one of claims 1 to 6, wherein the pH of the soapstock layer containing a γ-oryzanol salt is higher than 10 in step (A).

8. The method according to any one of claims 1 to 7, wherein, in step (B), the acid is added in such an amount that the pH of the soapstock layer becomes 10 or lower.

9. The method according to claim 8, wherein, in step (B), the acid is added in such an amount that the pH of the soapstock layer becomes 7 to 10.

10. The method according to any one of claims 1 to 9, wherein the acid is one or more kinds selected from the group consisting of hydrochloric acid, sulfuric acid, nitric acid, acetic acid, phosphoric acid, citric acid, lactic acid, malic acid, phytic acid, gluconic acid, tartaric acid, butyric acid, oxalic acid, fatty acid and deodorizer distillate.

11. The method according to any one of claims 1 to 10, further comprising a decolorization step and a deodorization step after step (B).

12. A γ-oryzanol-containing fat or oil producible by the method according to any one of claims 1 to 11.

13. The γ-oryzanol-containing fat or oil according to claim 12, containing 2 mass% or more of γ-oryzanol.

14. The γ-oryzanol-containing fat or oil according to claim 12 or 13, which is one or more kinds selected from the group consisting of rice bran oil, palm oil, rapeseed oil, soybean oil, sesame oil, corn oil, cotton seed oil, coconut oil, olive oil, palm oil, safflower oil, sunflower seed oil, almond oil, cashew oil, hazelnut oil, macadamia nut oil, mongongo oil, pecan oil, pine nut oil, walnut oil, camellia oil, tea seed oil, beef tallow, lard, chicken oil, horse oil and fish oil.

15. The γ-oryzanol-containing fat or oil according to claim 12 or 13, which is one or more kinds selected from the group consisting of mineral oil, squalene, lanolin, isopropyl isostearate, isostearyl isostearate, octyl isononanoate, ethyl oleate, ethyl oleate, octyl stearate, stearyl stearate, octyl palmitate, isopropyl palmitate, isopropyl myristate and octyldodecyl myristate.

【Fig. 1】

【Fig. 2】

## INTERNATIONAL SEARCH REPORT

| International application No. |
|---|
| PCT/JP2011/075662 |

A.  CLASSIFICATION OF SUBJECT MATTER
*A23D9/007*(2006.01)i, *A23K1/16*(2006.01)i, *A23L1/30*(2006.01)i, *C07J13/00*
(2006.01)i, *A23K1/00*(2006.01)n

According to International Patent Classification (IPC) or to both national classification and IPC

B.  FIELDS SEARCHED

Minimum documentation searched (classification system followed by classification symbols)
A23D9/007, A23K1/16, A23L1/30, C07J13/00, A23K1/00

Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)
JSTPlus(JDreamII), JST7580(JDreamII), FSTA/FOODLINE/FOODS ADLIBRA(DIALOG)

C.  DOCUMENTS CONSIDERED TO BE RELEVANT

| Category* | Citation of document, with indication, where appropriate, of the relevant passages | Relevant to claim No. |
|---|---|---|
| X | JP 2001-226693 A  (The Nisshin Oil Mills, Ltd.), 21 August 2001 (21.08.2001), claim 1; paragraphs [0005], [0015], [0016] (Family: none) | 12-15 |
| X | JP 2007-138067 A  (Ikko Chemical Co., Ltd.), 07 June 2007 (07.06.2007), claim 1 (Family: none) | 12-14 |
| X | JP 2007-124917 A  (Tsuno Food Industrial Co., Ltd.), 24 May 2007 (24.05.2007), claims 1, 2 (Family: none) | 12-14 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 24 January, 2012 (24.01.12) | 07 February, 2012 (07.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/075662 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2005-027505 A  (Toyo Seikan Kaisha, Ltd.), 03 February 2005 (03.02.2005), paragraphs [0082], [0087], [0088] (Family: none) | 12-15 |
| X | JP 2001-224309 A  (The Nisshin Oil Mills, Ltd.), 21 August 2001 (21.08.2001), claim 1; paragraph [0022] (Family: none) | 12-15 |
| A | JP 07-026288 A  (Tokyo Yushi Kogyo Kabushiki Kaisha), 27 January 1995 (27.01.1995), entire text (Family: none) | 1-15 |
| A | US 6197357 B1  (Carl W. Lawton), 06 March 2001 (06.03.2001), entire text (Family: none) | 1-15 |
| A | JP 2000-119682 A  (The Nisshin Oil Mills, Ltd.), 25 April 2000 (25.04.2000), entire text (Family: none) | 1-15 |
| A | VAN HOED Vera et al., Optimization of Physical Refining to Produce Rice Bran Oil with Light Color and High Oryzanol Content., J. Am. Oil Chem. Soc., 2010.10, Vol.87, p.1227-1234 | 1-15 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6340889 A
- JP 2002238455 A
- JP 2000119682 A
- JP 2009108145 A

**Non-patent literature cited in the description**

- The Standard Methods for the Analysis of Fats, Oils and Related Materials. Japan Oil Chemists' Society, 2003